(19) Europäisches Patentamt
European Patent Office
Office européen des brevets

(11) Veröffentlichungsnummer: **0 401 491**
**A2**

(12) # EUROPÄISCHE PATENTANMELDUNG

(21) Anmeldenummer: **90106860.1**

(22) Anmeldetag: **10.04.90**

(51) Int. Cl.5: **C07D 315/00, C07D 309/30, C07C 45/49, A61K 7/46**

(30) Priorität: **09.06.89 DE 3918881**

(43) Veröffentlichungstag der Anmeldung:
**12.12.90 Patentblatt 90/50**

(84) Benannte Vertragsstaaten:
**CH DE ES FR GB IT LI NL**

(71) Anmelder: **HÜLS AKTIENGESELLSCHAFT**
**Patentabteilung / PB 15 - Postfach 13 20**
**D-4370 Marl 1(DE)**

(72) Erfinder: **Keil, Thomas, Dr.**
**Siegerlandstrasse 27**
**D-4350 Recklinghausen(DE)**
Erfinder: **Schulze, Klaus, Dr.**
**Zum Seeblick 20**
**D-4358 Haltern(DE)**

(54) **Gemisch aus isomeren Alkylmethyltetrahydropyran-2-onen sowie ein Verfahren zu dessen Herstellung und Verwendung.**

(57)

2.1 Die Cyclopentanone der Formel (I)

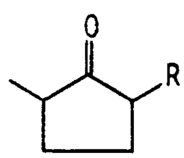

(I)

fallen als Nebenprodukte an. Es bestand daher die Aufgabe, aus diesen Verbindungen hochwertige Feinchemikalien zu synthetisieren.

2.2 Es werden hierzu neue Isomerengemische aus 6-Alkyl-3-methyl- und 3-Alkyl-6-methyltetrahydropyran-2-onen vorgeschlagen, die durch Baeyer-Villiger-Oxidation synthetisierbar sind. Diese Produkte weisen einen charakteristischen Eigengeruch auf.

2.3 Verwendung der neuen Isomerengemische von Alkylmethyltetrahydropyranonen als Riechstoffe.

EP 0 401 491 A2

# Gemisch aus isomeren Alkylmethyltetrahydropyran-2-onen sowie ein Verfahren zu dessen Herstellung und Verwendung

Die Erfindung betrifft ein Gemisch aus isomeren Alkylmethyltetrahydropyran-2-onen sowie ein Verfahren zu dessen Herstellung und Verwendung.

Aufgrund anderer chemischer Prozesse kann man die Verbindungen der allgemeinen Formel (I)

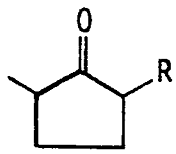

(I)

in größeren Mengen, z. B. nach der US-PS 2 995 607, herstellen.

Es bestand daher die Aufgabe, aus der billigen, leicht und in großen Mengen verfügbaren Chemikalie 2-Alkyl-5-methylcyclopentanon eine hochwertige Feinchemikalie zu synthetisieren.

Diese Aufgabe wurde durch das Synthesegemisch aus 6-Alkyl-3-methyl-und 3-Alkyl-6-methyltetrahydropyran-2-on gelöst.

Gegenstand der Erfindung sind daher Alkylmethyltetrahydropyran-2-one bestehend aus einem Gemisch von Verbindungen der Formel (II)

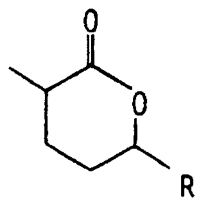

(II)

und Formel (III)

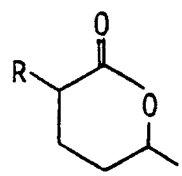

(III),

worin R = Alkylgruppe mit 2 bis 10 C-Atomen bedeutet.

Die Verbindungen der Formel (III) mit R = n-Propyl bzw. Ethyl sind literaturbekannt und lassen sich nach J. Colange und F. Guigues in Bull. Soc. Chim. Fr. 10, 3881-8, (1967), durch Verseifung von 5-Cyano-2-hydroxyoctan bzw. 5-Cyano-2-hydroxyheptan und anschließender intramolekularer Kondensation der Hydroxycarbonsäuren herstellen. Nach der DE-PS 28 18 244 ist bekannt, daß man aus 2,2,4-Trimethylcyclopentanon beziehungsweise 2,4,4-Trimethylcyclopentanon mittels Baeyer-Villliger-Oxidation zu 4,6,6-Trimethyltetrahydropyran-2-on beziehungsweise 4,4,6-Trimethyltetrahydropyran-2-on gelangt.

Es wurde nun überraschend gefunden, daß man aus den Ketonen der Formel (I) durch Bayer-Villiger-Reaktion zu dem hochwertigen Isomerengemisch der Pyran-2-one der Formeln (II) und (III) in hervorragenden Ausbeuten gelangt. Weiterhin ist überraschend, daß diese Isomerengemische äußerst gute Riechstoffeigenschaften aufweisen.

Die Herstellung des Gemisches der Alkylmethyltetrahydropyran-2-one im Verhältnis von 2 : 1 (oder 66 : 34) der Formeln (II) und (III) erfolgt in sehr guten Ausbeuten von 65 bis 99 % durch eine Baeyer-Villiger-Oxidation mittels einer Persäure. Als bevorzugte Persäure ist Peroxidischwefelsäure zu verwenden, die in situ aus Kaliumperoxodisulfat und Schwefelsäure gebildet wird.

Bevorzugt eingesetzte Pentanone sind diejenigen mit R = Alkylgruppe mit 2 bis 6 C-Atomen, wie z. B. 2-Methyl-5-n-propylcyclopentanon, so daß das bevorzugte Gemisch von (II) : (III) im Falle von R = n-Propyl

in einer Zusammensetzung von (II) : (III) = 2 : 1 in 99 % Ausbeute entsteht.

Die Synthesegemische der Alkylmethyltetrahydropyran-2-one stellen neben wertvollen Riechstoffkomponenten auch wertvolle Zwischenprodukte für pharmazeutische Wirkstoffe dar.

Durch ihren charakteristischen Eigengeruch können sie auch direkt als Riechstoffe Verwendung finden.

Beispiel 1 (Herstellungsbeispiel)

Zu einer Mischung von 180 ml konz. Schwefelsäure und 60 ml Wasser werden bei einer Temperatur von < 10 °C langsam unter Rühren 121,7 g (0,45 mol) Kaliumperoxodisulfat gegeben. Anschließend wird auf 15 °C erwärmt und weitere 200 ml Wasser zugegeben. Man kühlt auf 7 °C ab und tropft bei dieser Temperatur über einen Zeitraum von ca. 40 Minuten 33,7 g (0,24 mol) 2-Methyl-5-n-propylcyclopentanon zu. Anschließend wird auf Raumtemperatur erwärmt und über Nacht gerührt. Die Reaktionslösung wird in 600 ml Eiswasser gegeben und mit Ammoniumsulfat gesättigt. Man extrahiert mehrere Male mit Petrolether, vereinigt die Petroletherphasen und wäscht diese mit Natriumhydrogencarbonatlösung und Wasser neutral. Nach dem Trocknen über Natriumsulfat wird der Petrolether abgezogen und das Produkt destilliert. Man erhält 37,3 g (0,239 mol) = 99,4 % eines Gemisches von 3-Methyl-6-n-propyltetrahydropyran-2-on und 6-Methyl-3-n-propyltetrahydropyran-2-on im Verhältnis 66 : 34. Beide Komponenten liegen darüber hinaus jeweils als ein cis/trans-Gemisch im Verhältnis 1 : 1 vor.

Kennzahlen:
$n_D^{20}$ : 1,4531; Kp.: 81 - 82 °C / 0,2 mbar
GC/MS: MM = 156 (41, 56, 55, 42)

Beispiel 2 (Herstellungsbeispiel)

Es wird analog Beispiel 1 gearbeitet.

Ansatz:
240,0 ml konz. Schwefelsäure
80,0 ml Wasser
162,2 g (0,6 mol) Kaliumperoxodisulfat
260,0 ml Wasser
53,84 g (0,32 mol) 2-Methyl-5-n-pentylcyclopentanon
800,0 ml Eiswasser

Man erhält nach der Destillation 38,4 g (= 0,21 mol) = 65,1 % eines Gemisches von 3-Methyl-6-n-pentyltetrahydropyran-2-on und 6-Methyl-3-n-pentyltetrahydropyran-2-on im Verhältnis 66 : 34. Die Komponen ten liegen jeweils als cis/trans-Isomere im Verhältnis 1 : 1 vor.

Kennzahlen:
$n_D^{20}$ : 1,4562; Kp. = 98 °C/0,2 mbar;
GC/MS: MM = 184 (56, 55, 114, 41)

Beispiel 3 (Verwendungsbeispiel)

Das nach Beispiel 1 dargestellte Gemisch weist einen intensiven Geruch nach gerösteten Nüssen auf.

**Ansprüche**

1. Alkylmethyltetrahydropyran-2-one bestehend aus einem Gemisch von Verbindungen der Formel (II)

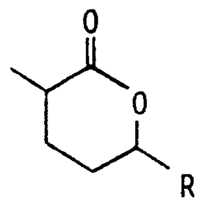

(II)

und Formel (III)

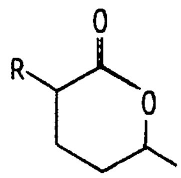

(III),

worin R = Alkylgruppe mit 2 bis 10 C-Atomen bedeutet.

2. Alkylmethyltetrahydropyran-2-one bestehend aus einem Gemisch von Verbindungen der Formeln (II) und (III) nach Anspruch 1,
dadurch gekennzeichnet,
daß das Verhältnis von (II) : (III) 2 : 1 beträgt.

3. Alkylmethyltetrahydropyran-2-one bestehend aus einem Gemisch von Verbindungen der Formeln (II) und (III) nach Anspruch 1,
dadurch gekennzeichnet,
daß R = Alkylgruppe mit 2 bis 6 C-Atomen bedeutet.

4. Verfahren zur Herstellung von Alkylmethyltetrahydropyran-2-onen bestehend aus einem Gemisch von Verbindungen der Formeln (II) und (III) nach den Ansprüchen 1 bis 3,
dadurch gekennzeichnet,
daß man Verbindungen der allgemeinen Formel (I)

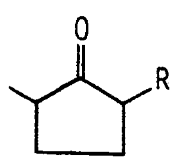

(I)

wobei R = Alkyl mit 2 bis 10 C-Atomen bedeutet,
einer Baeyer-Villiger-Reaktion bei Temperaturen $T < 10\ ^{\circ}C$ mittels Persäure unterwirft.

5. Verfahren zur Herstellung von Alkylmethyltetrahydropyran-2-onen bestehend aus einem Gemisch von Verbindungen der Formeln (II) und (III) nach Anspruch 4,
dadurch gekennzeichnet,
daß man als Persäure in situ aus Kaliumperoxodisulfat und Schwefelsäure hergestellte Peroxidischwefelsäure verwendet.

6. Verfahren zur Herstellung von Alkylmethyltetrahydropyran-2-onen bestehend aus einem Gemisch von Verbindungen der Formeln (II) und (III) nach Anspruch 4,
dadurch gekennzeichnet,
daß man als Verbindung der allgemeinen Formel (I) Verbindungen, bei denen R = Alkylgruppe mit 2 bis 6 C-Atomen bedeutet, verwendet.

7. Verwendung von Alkylmethyltetrahydropyran-2-onen bestehend aus einem Gemisch von Verbindungen der Formeln (II) und (III) nach den Ansprüchen 1 bis 3 als Riechstoff.